# EUROPÄISCHE PATENTSCHRIFT

(11) **EP 1 501 562 B1**
(45) Veröffentlichungstag und Bekanntmachung des Hinweises auf die Patenterteilung: **25.05.2011**
(21) Anmeldenummer: 03727402.4
(22) Anmeldetag: 30.04.2003
(51) Int. Cl.: A61L 29/12

(54) **KATHETERSCHLAUCH**
CATHETER TUBE
TUYAU DE CATHETER

(30) Priorität: 08.05.2002 DE 10220409
(43) Veröffentlichungstag der Anmeldung: 02.02.2005
(73) Patentinhaber: RAUMEDIC AG, 95233 Helmbrechts (DE); B. Braun Melsungen AG, 34209 Melsungen (DE)
(72) Erfinder: DOLLA, Andreas, 95032 Hof (DE); GÖBEL, Udo, 34212 Melsungen-Kirchhof (DE)
(74) Vertreter: Hofmann, Matthias
(86) Internationale Anmeldenummer: PCT/EP2003/004516
(87) Internationale Veröffentlichungsnummer: WO 2003/094989

(56) Entgegenhaltungen:
- EP-A- 0 010 757
- EP-A- 0 422 632
- EP-A- 0 737 487
- WO-A-90/01345
- WO-A-94/01160
- WO-A-03/024498
- DE-A- 10 122 203
- US-A- 4 563 181

## Beschreibung

Die Erfindung betrifft einen Katheterschlauch mit verbesserten Eigenschaften.

Für die Anwendung im Bereich Regionalanästhesie sind Katheterschläuche als Monoschläuche, mit und ohne Röntgenkontraststreifen, Stand der Technik.

Hierzu werden Werkstoffe, wie PA, PUR, PTFE, PE, PVC und Silikon eingesetzt, die auf Grund ihrer Eigenschaften - bezogen auf die Regionalanästhesie - folgendermaßen einzuordnen sind:

| Werkstoff | Chem. Beständigkeit gegenüber Anästhetikas | Biegesteifigkeit | Knickstabilität | Reißfestigkeit | Dehnung | Transparenz |
|---|---|---|---|---|---|---|
| PVC | - | o | ○ | ○ | o | + |
| PE | + | o | - | + | + | - |
| Silicon | + | - | + | O | + | - |
| PA | + | + | o/+ | + | o | + |
| PTFE | + | - | - | + | o | - |
| PUR | + | o | + | + | + | + |
| FEP | + | - | - | + | + | - |

Weitere Informationen zur Indikation, Applikation und medizinische Anforderungen finden sich in den nachfolgend aufgeführten Literaturstellen:

| | |
|---|---|
| Niesel Hans Christoph | Regionalanästhesie Lokalanästhesie Regionale Schmerztherapie, Georg Thieme Verlag Stuttgart New York, 1994, 291 - 350 |
| | |
| Hahn B. H. | Regionalanästhesie |
| NcQuillan M. | Ullstein Medical Verlagsgesellschaft mbH & Co., Wiesbaden, |
| Sheplock G. J. | 1999, 209 - 215 |
| | |
| Jankovic Danilo, | Regional Nerve Blocks, |
| Wells Christopher | Blackwell Science Berlin Vienna, 2001, 243 - 252 |
| | |
| Moor D. C. | Regional Block |
| | Charles C Thomas, Springfield ■ Illinois ■ USA, Fourth Edition |
| | 1979, 427 - 438 |
| Saint - Maurice | Regional Anaesthesia in Children |
| Schulte Steinberg | Appleton & Lange / Mediglobe, Norwalk / San Meteo / Fribourg, 1990, 106 -109 |
| | |
| Scott D. B. | Handbook of Epidural Anaesthesia and Analgesia, Schulz Medical Information Copenhagen Denmark, 1985; 86 - 99 |

Nachfolgend sei an den Beispielen Biegesteifigkeit, Vorschiebbarkeit, Rückstellkraft und Knickbeständigkeit, also Eigenschaften, die für die Regionalanästhesie von Bedeutung sind, erläutert, wie sich die Werkstoffeigenschaften auf die Applikation des Katheterschlauches beim Patienten auswirken können.

### Biegesteifigkeit

Ist der Katheterschlauch zu weich, lässt er sich schlecht im Epiduralraum platzieren, speziell bei schwierigen anatomischen Verhältnissen. Des Weiteren besteht das Risiko von Fehllagen, speziell einer seitlichen Aberration bis hin zu einer Schlingen- und Knotenbildung.

Ist der Katheterschlauch zu hart, bedeutet dies ein hohes Risiko von Intravasal- und Intrathekallagen sowie Nervenirritationen, die reversibel als auch irreversibel sein können.

### Knickstabilität

Eine geringe Knickstabilität birgt die Gefahr eines Verschlusses des Katheterschlauches, mit den Konsequenzen, dass der Patient nicht oder nur unzureichend mit Analgetika versorgt wird oder es zu Pumpenalarm kommen kann, wenn Infusionspumpen zur Applikation des Anästhetikums eingesetzt werden.

### Vorschiebbarkeit

Besitzt ein Katheterschlauch eine unzureichende Vorschiebbarkeit, lässt er sich schlecht bzw. überhaupt nicht durch die Epiduralkanüle in den Epiduralraum platzieren. Dies führt zu Verlängerungen der Applikationszeit und eine unnötige Patientenbelastung oder zum Ausweichen auf eine andere Anästhesietechnik.

### Rückstellkraft

Eine unzureichende Rückstellkraft des Katheterschlauches führt zu Fehllagen, bedingt durch eine zu geringe bzw. fehlende Neigung des Katheterschlauches, seine ursprüngliche Lage vor der Auslenkung zu erreichen.

Betrachtet man den aktuellen Stand der Technik, ist der Umstand besonders nachteilig, dass produktionsbedingt die mechanischen Eigenschaften der Rohstoffe teilweise erheblich schwanken. Dies drückt sich vor allem in den Shorehärteangaben der Rohstofflieferanten aus. Üblich sind Toleranzen von ± 4 Einheiten. Beispielsweise kann bei einem PUR der angegebenen Shorehärte 60 D diese in der Praxis zwischen 56 D und 64 D liegen, was sich signifikant auf die mechanischen Eigenschaften des Katheterschlauches, beispielsweise die Biegesteifigkeit, auswirkt, mit den oben genannten Negativpunkten. Die mechanischen Eigenschaften des Kathetermonoschlauches lassen sich also nicht reproduzierbar und auch nicht gezielt bzw. quasi stufenlos einstellen.

Weiterhin hat der Stand der Technik den Nachteil, dass die medizinisch außerordentlich erwünschte Eigenschaft eines Katheters, nämlich bei der Applikation, also beim Einführen in den Körper, möglichst steif, für die Zeit des Verweilens im Körper zur Schonung des Gewebes und zur Vermeidung von Entzündungen jedoch möglichst flexibel zu sein, mit Monoschläuchen nur sehr begrenzt realisierbar ist.

Die WO 90/01345 A beschreibt einen Katheter zum Einsatz in Blutgefäßen. Ein Katheterschlauch hierfür hat eine dem Lumen zugewandte Innenbeschichtung, für die als Materialbeispiel ein Copolymer aus Polyurethan und Polyvinylpyrrolidon angegeben ist. Ferner weist der Katheterschlauch eine Innenschicht und eine Außenschicht auf, die aus PEBA gefertigt sind. Optional hat der Katheter auch eine Außenbeschichtung, die aus dem gleichen Material sein kann wie die Innenbeschichtung.

Die EP 0 010 757 A1 beschreibt einen Katheter mit wendelförmig angeordneten Kontraststreifen.

Die WO 94/01160 A1 beschreibt einen Anästhesie-Katheterschlauch mit einem härteren Innenschlauch, unter anderem aus Polyamid, und einem im Bereich der Katheterspitze über den Innenschlauch überstehenden weicheren Außenschlauch, unter anderem aus Polyurethan. Ein entsprechender Katheterschlauch mit einem Polyamid-Innenschlauch und einem Polyurethan-Außenschlauch ist beispielsweise auch aus der DE 40 32 869 A1 bekannt.

Die Aufgabe der Erfindung ist es, einen Katheterschlauch mit folgenden Eigenschaften bereitzustellen:
- Konstantes bzw. spezifizierte Eigenschaftsniveau, insbesondere bei den mechanischen Eigenschaften
- Hohe Steifigkeit beim Vorschieben des Katheterschlauches, was letztlich eine gute Vorschiebbarkeit ergibt
- Flexibles Verhalten zur Minimierung von Intravasal- und intratekallagen sowie von Nervenirritationen
- Hohe Knickstabilität
- Reduzierung der Steifigkeit nach Platzierung des Katheterschlauches zur Minimierung von Hämatombildungen
- Möglichst geringe Absorption gegenüber den üblichen Lokalanästhetikas
- Beibehaltung der mechanischen und biologischen Eigenschaften bei der Langzeitapplikation

Nachfolgend werden die Überlegungen beschrieben, die zur Lösung der Aufgabe führten:
Für die Herstellung von schlauchförmigen Extrudaten, gerade auch für die Medizin, werden Homo-Polymere, meist jedoch Co-Polymere oder Polymermischungen, eingesetzt. Reichen jedoch diese standardmäßig käuflichen Werkstoffe in ihrem Eigenschaftsspektrum nicht aus, ist meist ein erheblicher, rezepturseitiger Aufwand nötig, um die gewünschte Eigenschaftsmodifikation zu erzielen.

In Fällen, bei denen die Schlauch-Innenfläche wesentlich andere Eigenschaften aufweisen muss als die Außenfläche - beispielsweise wenn die äußere Schicht eine gute Verklebbarkeit, die innere Schicht eine gute Beständigkeit gegenüber Lokalanästhetika aufweisen soll - ist eine Rezeptierung jedoch nicht mehr zielführend. Hier behilft man sich gerne mit Schichtverbunden von Werkstoffen unterschiedlicher Eigenschaften. Die makroskopischen Eigenschaften solcher Schichtverbunde, wie Steifigkeit oder Biegefestigkeit, werden dabei in der Regel durch die Summe der anteiligen Werkstoffeigenschaften bestimmt.

Beispielsweise beschreibt die US 4 385 632 einen Schichtverbund für einen Angeographiekatheter. Dieser Katheterschlauch verfügt über eine weiche, atraumatische Spitze ausschließlich aus Polyurethan, einen Übergangsbereich und einen Hauptschlauch, bestehend aus einer Polyamid-Innenschicht und einer Polyurethan-Außenschicht im annähernden Wanddicken-Verhältnis 50 : 50.

Ziel dieser Ausführung in Form eines Schichtverbundes ist es, folgende Anforderungen zu erfüllen:
- Eine weiche, atraumatische, unverstärkte Polyurethanspitze zu ermöglichen,
- diese fest auf dem Katheterschlauch zu verankern,
- eine entsprechend hohe Festigkeit des Katheters bei geringer Wandstärke zu gewährleisten. Diese hohe Festigkeit ist erforderlich, da das Röntgenkontrastmittel mit hohem Druck (bis zu 1200 psi) appliziert wird und dabei ein Platzen des Katheters sicher vermieden werden muss.

Dem gegenüber zielt die vorliegende Erfindung darauf ab, für den Bereich Regionalanästhesie Kathetereigenschaften zu erreichen, wie sie in der Aufgabenstellung oben beschrieben sind. Das ist nur möglich, wenn über die Werkstoffeigenschaften der einzelnen Schichten neue Eigenschaftskombinationen erreicht werden, die über die Summe der Einzeleigenschaften deutlich hinaus gehen. Dies sollte nur dann erzielbar sein, wenn die Systeme einen gewissen synergistischen Effekt zeigen, darüber hinaus die eingesetzten Werkstoffe für den jeweiligen Anwendungsfall exakt abgestimmt sind.

Die Aufgabe konnte überraschenderweise dadurch gelöst werden, dass der Katheterschlauch aus einem Schichtverbund von PUR-Außenschicht mit PA-Innenschicht besteht. Dabei ist das Polyamid ausgewählt aus der Gruppe Polyamid 11, Polyamid 12 oder Polyetherblockamid, das Polyurethan aus der Gruppe Polyetherpolyurethan oder Polyesterpolyurethan.

Nachfolgend werden die Eigenschaften der Erfindung näher erläutert.

Wie schon erwähnt, ist es aus medizinischer Sicht außerordentlich erwünscht, dass ein Katheterschlauch beim Einführen in die Körper möglichst steif, für das Verweilen im Körper jedoch möglichst flexibel ist. Diese Eigenschaften, die sich zunächst auszuschließen scheinen, sind überraschenderweise über die erfindungsgemäße Werkstoffkombination zu erreichen: PUR wird in erster Linie durch Temperatureinwirkung flexibel und weich. Dieser Vorgang vollzieht sich innerhalb von wenigen Minuten bereits bei Körpertemperatur. Beispielsweise sinkt eine Ausgangsshorehärte von 60 D bei 37° C innerhalb von ca. 5 Minuten auf 50 D. Dem gegenüber wird PA ausschließlich in Folge von Feuchtigkeitsaufnahme in seiner Flexibilität beeinflusst, wobei dieser Vorgang im Vergleich zu PUR einen wesentlich längeren Zeitraum benötigt.

Besonders vorteilhaft ist, dass je nach den relativen Wandstärken PUR zu PA und den jeweils eingesetzten Shorehärten sich somit die für die Platzierung des Katheterschlauches erforderliche Steifigkeit einerseits und die für das Verweilen im Körper nötige Flexibilität andererseits exakt einstellen lässt. Weiterhin lassen sich durch die relativen Schichtstärken der beiden Polymere und die Wahl von Standardtypen mit unterschiedlichen Shorehärten nicht nur die Flexibilität/Weichheit, sondern auch weitere, für die Anwendung der Katheterschläuche wichtige Eigenschaften gezielt einstellen, nämlich die Biegesteifigkeit, Knickstabilität, Verschiebbarkeit und das Rückstellverhalten. Diese Möglichkeit ist bei den aktuell verwendeten Anästhesiekathetern auf Basis von Monoschläuchen gemäß dem Stand der Technik nicht gegeben.

Erfindungsgemäß lassen sich auch, vorteilhafterweise, die gewünschten Eigenschaften des Katheterschlauches bei Schwankungen der Rohstoffqualität durch Variation der Schichtdicken des koextrudierten Schlauches weitgehend gewährleisten. Darüber hinaus ist es durch den Einsatz verschiedener, gängiger Standard-Rohstoffe mit unterschiedlichen mechanischen Eigenschaften möglich, die Gesamteigenschaften des Verbundschlauches erheblich, jedoch in einfacher Weise, ändern - ohne dass spezielle Rezeptur-Abmischungen erforderlich sind.

Diese Rezeptur-Abmischungen sind jedoch erfindungsgemäß zusätzlich möglich und können eine weitere Eigenschaftsoptimierung bzw. Feinabstimmung der Kathetereigenschaften bewirken.

Für die Anwendung in der Regionalanästhesie haben sich drei Biegesteifigkeitsbereiche für die unterschiedlichen Einsatzgebiete als optimal herausgestellt:

### Biegesteifigkeitsbereich

I. 80 - 90 mN
II. 170 - 180 mN
III. 300 - 335 mN

Grundsätzlich lassen sich bei einer medizinisch bedingten Gesamtwandstärke die Parameter
- Wandstärke Innenschlauch
- Wandstärke Außenschlauch
- Shorehärte des PA-Innenschlauches
- Shorehärte des PUR-Außenschlauches
so auswählen, dass jeweils der gewünschte makroskopische Biegesteifigkeitsbereich des Katheterschlauches erreicht wird.

Setzt man beispielsweise für einen Katheterschlauch mit der Abmessung 0,85 x 0,45 mm und den Biegesteifigkeitsbereich II für den Innenschlauch eine PA-Type mit einer Shorehärte von 74 D und eine PUR-Type von Shore A 85 ein, ergibt sich eine Wandstärke des Innenschlauches von 0,08 mm und eine Wandstärke des Außenschlauches von 0,12 mm, also eine Wandstärkenverteilung von 40 % PA zu 60 % PUR.

Erfindungsgemäß bevorzugt sind jedoch Wandstärkenverteilungen PA zu PUR von annähernd 50 : 50 bis 70 : 30, da so nicht nur die erwünschte Biegesteifigkeit, sondern auch ein Optimum bezüglich Knickstabilität, Vorschiebbarkeit und Rückstellverhalten für die meisten Anwendungen erreicht wird.

Mit den erfindungsgemäßen Verbundschläuchen aus einer Kombination PA-Innenschlauch in Verbindung mit einem PUR-Außenschlauch ist es also möglich, nicht nur die aktuellen Eigenschaften der PA-Monoschläuche zu erreichen, sondern darüber hinaus deutliche Verbesserungen in Qualität und Eigenschaften, wie dem folgenden Ausführungsbeispiel zu entnehmen ist.

Die Erfindung soll anhand eines Anästhesiekatheters ohne Röntgenkontraststreifen näher erläutert werden.

Dieser verfügt im Beispiel über die Abmessung 0,85 x 0,45 mm, also eine Außenwandstärke von 0,85 mm, einen Innendurchmesser von 0,45 mm, und damit eine Wandstärke von 0,20 mm.

Die Wandstärkenverteilung beträgt 30 %, PUR-Außenschicht, entsprechend 0,06 mm, und PA-Innenschicht 70 %, entsprechend 0,14 mm.

Betrachtet man die Abhängigkeiten der Biegesteifigkeit des Gesamtsystems, also des Katheterschlauches, in Abhängigkeit von den Shorehärten der jeweiligen Werkstoffe, ergibt sich folgendes Ergebnis:

| Beispiel | Werkstoffkombination | Biegesteifigkeit |
|---|---|---|
| 1 | PA70D/PA70D | 110 - 130mN |
| 2 | PA 70D / PUR 55D | 60 - 65 mN |
| 3 | PA 70D / PUR 60 D | 80 - 90 mN |
| 4 | PA 70D / PUR 65 D | 130 - 150 mN |
| 5 | PA70D/PUR68D | 170 - 180mN |
| 6 | PA 70D / PUR 70 D | 200 - 220 mN |

Die Tabelle zeigt also beispielsweise, dass man unter Beibehaltung der Shore - Härte des reinen PA-Katheters die Biegesteifigkeit um 50 % erhöhen kann, wenn man anstelle von PA eine Außenschicht aus PUR wählt (Beispiel 1 zu Beispiel 6). Dabei tritt gleichzeitig der bereits erwähnte Effekt auf, dass der erfindungsgemäße Katheterschlauch kurze Zeit nach der Applikation unter Einwirkung der Körpertemperatur wesentlich weicher ist, als der reine PA-Katheterschlauch.

Die folgende Tabelle zeigt für eine zweite Abmessung des Katheterschlauches, hier 1,05 mm x 0,60 mm, die Abhängigkeit der Biegesteifigkeit in Abhängigkeit von der PUR-Shorehärte, wobei das Verhältnis PA-Innenschicht zur PUR - Außenschicht mit 70 % zu 30 % unverändert blieb.

| Beispiel | Werkstoffkombination | Biegesteifigkeit |
|---|---|---|
| 7 | PA 70D/PUR 60D | 250 - 290 mN |
| 8 | PA 70D/PUR 68D | 300 - 335 mN |
| 9 | PA 70D/PUR 70D | 410 - 430 mN |

Interessant ist der Vergleich der Beispiele 6 und 9, aus denen hervorgeht, wie stark - bei einer jeweils eingestellten Shorehärte von 70 D - die Biegesteifigkeit des Katheterschlauches von der Abmessung abhängt.

## Patentansprüche

1. Anästhesie-Katheterschlauch aus Polymeren für die Regionalanästhesie, der aus einem Schichtverbund aus Polyamid und Polyurethan besteht, wobei die dem Lumen zugewandte Innenseite aus einer Polyamidschicht, die Außenseite aus einer Polyurethanschicht besteht, **dadurch** gekenntzeichnet, dass der Schichtverbuud ein Koextrudat ist und eine Schichtdickenverteilung PA-Innenschicht zu PUR-Außenschicht von 50:50 bis 70:30 aufweist.

2. Katheterschlauch nach Anspruch 1, **dadurch gekennzeichnet, dass** das Polyamid ausgewählt ist aus der Gruppe Polyamid 11, Polyamid 12 oder Polyetherblockamid.

3. Katheterschlauch nach Anspruch 1, **dadurch gekennzeichnet, dass** das Polyurethan ausgewählt ist aus der Gruppe Polyetherpolyurethan oder Polyesterpolyurethan.

4. Katheterschlauch nach einem der vorhergehenden Ansprüche, **dadurch gekennzeichnet, dass** die Innen- und/oder Außenschicht über einen oder mehrere Röntgenkontraststreifen verfügt.

## Claims

1. Anaesthetic catheter tube of polymers for regional anaesthesia, the tube consisting of a multi-layer composite of polyamide and polyurethane, with the inside facing the lumen consisting of a polyamide layer and the outside consisting of a polyurethane layer, **characterized in that** the multi-layer composite is a coextrudate and has a layer thickness distribution PA internal layer to PUR external layer of 50:50 to 70:30.

2. Catheter tube according to claim 1, **characterized in that** the polyamide is selected from the group of polyamide 11, polyamide 12 or polyether block amide.

3. Catheter tube according to claim 1, **characterized in that** the polyurethane is selected from the group of polyether polyurethane or polyester polyurethane.

4. Catheter tube according to one of the preceding claims, **characterized in that** the internal and/or external layer is provided with one or several X-ray contrast lines.

## Revendications

1. Tuyau de cathéter d'anesthésie en polymère pour l'anesthésie loco-régionale, qui est constitué d'un composite de polyamide et de polyuréthane, le côté intérieur tourné vers le lumen étant constitué d'une couche de polyamide, la couche extérieure étant constituée d'une couche de polyuréthane, **caractérisé en ce que** le composite est un co-extrudat et présente une répartition des épaisseurs de couche de la couche intérieure PA à la couche extérieur PUR de 50:50 à 70:30.

2. Tuyau de cathéter selon la revendication 1, **caractérisé en ce que** le polyamide est choisi dans le groupe comprenant le polyamide 11, le polyamide 12 ou le polyéther-bloc-amide.

3. Tuyau de cathéter selon la revendication 1, **caractérisé en ce que** le polyuréthane est choisi dans le groupe comprenant le polyéther-polyuréthane ou le polyester-polyuréthane.

4. Tuyau de cathéter selon l'une des revendications précédentes, **caractérisé en ce que** la couche intérieure et/ou la couche extérieure disposent d'une ou plusieurs bandes de contraste aux rayons X.
